# EUROPEAN PATENT APPLICATION

(11) **EP 4 151 209 A1**
(43) Date of publication of application: **22.03.2023**
(21) Application number: 20935665.8
(22) Date of filing: 15.05.2020
(51) Int. Cl.: A61K 31/4172, C07C 229/36, C07C 227/12, C07D 213/38, C07D 307/52

(54) **AMINO ACID COMPOSITION AND METHOD FOR CATALYTIC SYNTHESIS OF AMINO ACID BY MEANS OF ENERGY RADIATION**

(71) Applicant: Beijing Guanghe Original Technology Co., Ltd., Beijing 100080 (CN)
(72) Inventor: WANG, Cong, Beijing 100080 (CN); HUO, Haibin, Beijing 100080 (CN)
(74) Representative: Barrow, Nicholas Martin
(86) International application number: PCT/CN2020/090684
(87) International publication number: WO 2021/227077

(57) **Abstract**

The present invention provides an amino acid composition, and a method for producing amino acids by means of energy irradiation, the method comprises contacting a nanostructure catalyst with at least one nitrogen-containing source, at least one hydrogen-containing source and at least one carbon-containing source, and irradiating the nanostructure catalyst, the nitrogen-containing source, the hydrogen-containing source and the carbon-containing source with energy, to produce the amino acids.

## Description

### FIELD OF THE INVENTION

The present invention relates to an amino acid composition comprising amino acid essential to human body and a method for catalytically synthesizing amino acids by means of energy irradiation, which belongs to the field of sequestration and utilization of carbon dioxide and nitrogen fixation reaction.

### BACKGROUND

The greenhouse effect caused by massively increased carbon dioxide is a great threat to environment, climate and ecology of all humanity. Carbon dioxide capture, utilization and sequestration technologies are getting more and more attentions, and a mass of research projects have been carried out concerning such technologies by the worldwide scientists. Artificial photosynthesis technology is one of the most promising technologies, which converts carbon dioxide into organic compound forms such as hydrocarbons, alcohols and the like by means of solar energy, so as to achieve sequestration and utilization of carbon dioxide, and energy recycling.

Amino acids, as important vital substances, have practical value in medicine, and have been widely applied in fields such as biochemistry, medicine, nutriology and even industrial manufacture. At present, artificial amino acids are mainly produced by biological techniques or extracted from organisms, a method for effectively catalytically synthesizing amino acids has not been developed. The eight kinds of amino acids essential to human body include isoleucine, leucine, phenylalanine, lysine, threonine, valine, methionine and tryptophan.

Plasmonic catalysts could immensely enhance local energy on surfaces of a nanostructure due to the plasmon effect. Accordingly, in a case of the overall reaction conditions are mild, the catalytic reaction could be efficiently promoted such that it is possible to conduct a reaction that could not be conducted under normal temperature and normal pressure. Nitrogen and carbon dioxide could be converted to amino acids, by utilizing plasmonic catalyst.

### SUMMARY OF THE INVENTION

The present invention discloses an amino acid composition comprising amino acids essential to human body, and a new artificial photosynthesis technology, which provided a unique method for producing amino acids by utilizing CO₂ (and/or CO) and N₂ from industrial flue gas or atmosphere, by means of light irradiation and/or heat irradiation, in the presence of a nanostructure catalyst.

One aspect of the present invention is an amino acid composition comprising amino acids essential to human body, which comprises isoleucine, leucine, phenylalanine, lysine, threonine and valine, and preferably optionally comprises methionine and tryptophan.

In certain embodiments, the amino acid composition comprises 0.36-0.44 part by mole of isoleucine, 0.20-0.28 part by mole of leucine, 0.20-0.27 part by mole of phenylalanine, 0.008-0.32 part by mole of lysine, 0.02-0.14 part by mole of threonine, 0.02-0.35 part by mole of valine, 0-0.04 part by mole of methionine and 0-0.04 part by mole of tryptophan.

In certain embodiments, the amino acid composition further comprises glycine, alanine and cysteine.

In certain embodiments, the amino acid composition further comprises 0.12-0.71 part by mole of glycine, 0.30-0.62 part by mole of alanine and 0.14-0.23 part by mole of cysteine.

In certain embodiments, the amino acid composition further comprises serine, glutamine, proline, asparagine, aspartic acid, glutamate and tyrosine. In preferred embodiments, the amino acid composition preferably comprises water as solvent.

In preferred embodiments, the amino acid composition is substantially consisting of isoleucine, leucine, phenylalanine, lysine, threonine, valine, methionine and tryptophan.

Another aspect of the present invention is a method for producing amino acids by energy irradiation, comprising:
contacting a nanostructure catalyst with at least one nitrogen-containing source, at least one hydrogen-containing source and at least one carbon-containing source, and
irradiating the nanostructure catalyst, the nitrogen-containing source, the hydrogen-containing source and the carbon-containing source with energy, to produce amino acids, wherein
the nanostructure catalyst comprises at least one first component and at leas one second component.

In certain embodiments, the energy irradiation is at least one selected from light irradiation and heat irradiation.

In certain embodiments, a distance between the first component and second component is 200 nm or less, preferably 100 nm or less, and most preferably the first component and the second component are in close contact with each other.

In certain embodiments, the nanostructure catalyst comprises one chemical element as both the first component and the second component, or comprises two or more chemical elements, alloys, or compounds each as the first component or the second component.

In certain embodiments, the nanostructure catalyst is produced by physically mixing the at least one first component and the at least one second component.

In certain embodiments, the nanostructure catalyst provides the at least one first component and the at least one second component in one nanostructure.

In preferred embodiments, the first component is selected from a group consisting of Co, Fe, Al, Ag, Au, Pt, Cu, Ni, Zn, Ti, Mn, C, Pd, Ru and alloys of two or more chemical elements thereof, and preferably Ru, Co, Fe or C.

In preferred embodiments, the second component is selected from a group consisting of Co, Fe, Ru, Rh, Os, Ir, La, Ce, Cu, Ni, Ti, Mo, Bi, V, C and oxides, sulfites, carbides, hydroxides, chlorides, carbonates and bicarbonates thereof, and preferably Co, C or MnO₂.

In preferred embodiments, the nanostructure catalyst is a Co catalyst, a Co-Ru catalyst, a Fe/C catalyst or Fe/C/MnO₂ catalyst.

In certain embodiments, the aforementioned amino acid compositions could be produced via the method.

In certain embodiments, the nanostructure is about 1 nm to about 1000 nm, preferably about 70 nm to about 1000 nm, about 100 nm to about 800 nm, about 200 nm to 500 nm in at least one dimension of length, width and height.

In certain embodiments, the nanostructure each independently is about 1 nm to about 3000 nm in length, width or height, preferably about 100 nm to about 3000 nm, about 500 nm to about 2500 nm, or about 1000 nm to about 2000 nm in length, and/or about 1 nm to about 1000 nm, about 70 nm to about 1000 nm, about 100 nm to about 800 nm, or about 200 nm to about 500 nm in width or height, or
the nanostructure each independently has an aspect ratio of about 1 to about 20, and preferably an aspect ratio of about 1 to about 10, or about 2 to about 8.

In certain embodiments, the nanostructure catalyst each independently has a shape of spherical, spike, flake, needle, grass, cylindrical, polyhedral, 3D cone, cuboidal, sheet, hemispherical, irregular 3D shape, porous structure or any combinations thereof.

In certain embodiments, a plurality of the nanostructures are arranged in a patterned configuration, and preferably in a plurality of layers, on a substrate, or a plurality of the nanostructure are randomly dispersed in a medium.

In certain embodiments, the energy irradiation allows the reaction progresses at a temperature between about 20°C to about 300°C, preferably about 30°C to about 300°C, about 40 °C to about 300 °C, about 80 °C to about 300°C, about 100°C to about 280°C, about 110°C to about 270°C.

In certain embodiments, the reaction is initiated by means of light irradiation or heat irradiation, and the reaction is continued to progress by means of light irradiation or heat irradiation, wherein
a power of the light irradiation is 200-1500 W/m², preferably 200-1000 W/m², and most preferably 500-1000 W/m².

In certain embodiments, the temperatures of the nanostructure catalyst, the nitrogen-containing source, hydrogen-containing source and the carbon-containing source are raised by the light irradiation, and preferably the light irradiation is the sole source for rising the temperatures.

In certain embodiments, the nitrogen-containing source is selected from a group consisting of N₂, air, ammonia, nitrogen oxides, nitro compounds and any combination thereof, or air, industrial flue gas, exhausts or emissions comprising one or more of these nitrogen-containing sources, and preferably N₂.

In certain embodiments, the hydrogen-containing source is selected from a group consisting of water, H₂, C₁₋₄ hydrocarbons and any combination thereof, or air, industrial flue gas, exhausts or emissions comprising one or more of these hydrogen-containing sources, and preferably water.

In certain embodiments, the carbon-containing source is selected from a group consisting of CO₂, CO, C₁₋₄ hydrocarbons, synthesis gas, bicarbonate salts and any combination thereof, or air, industrial flue gas, exhausts or emissions comprising one or more of these carbon-containing sources, and preferably CO₂ or CO.

### BRIEF DESCRIPTION OF THE DRAWINGS

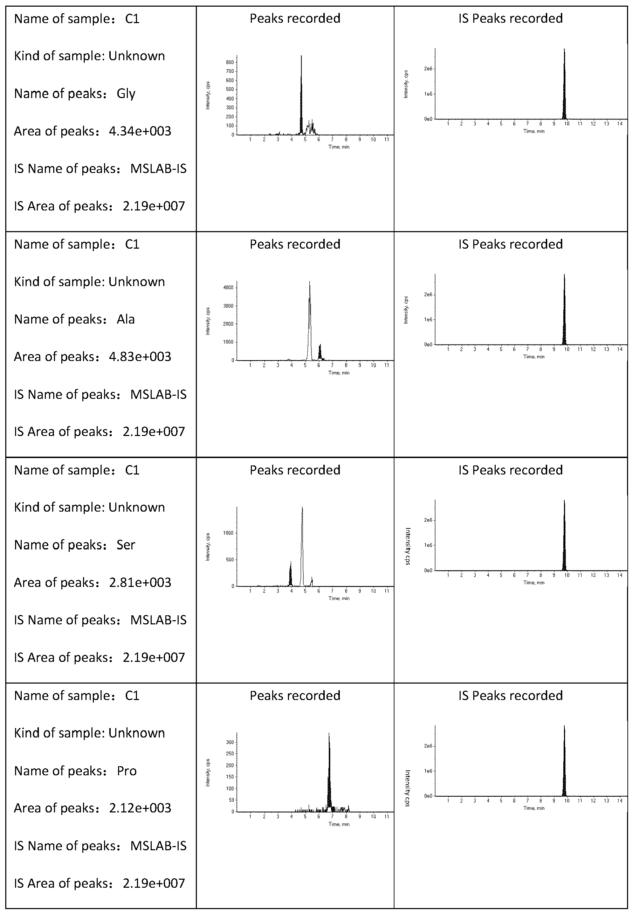

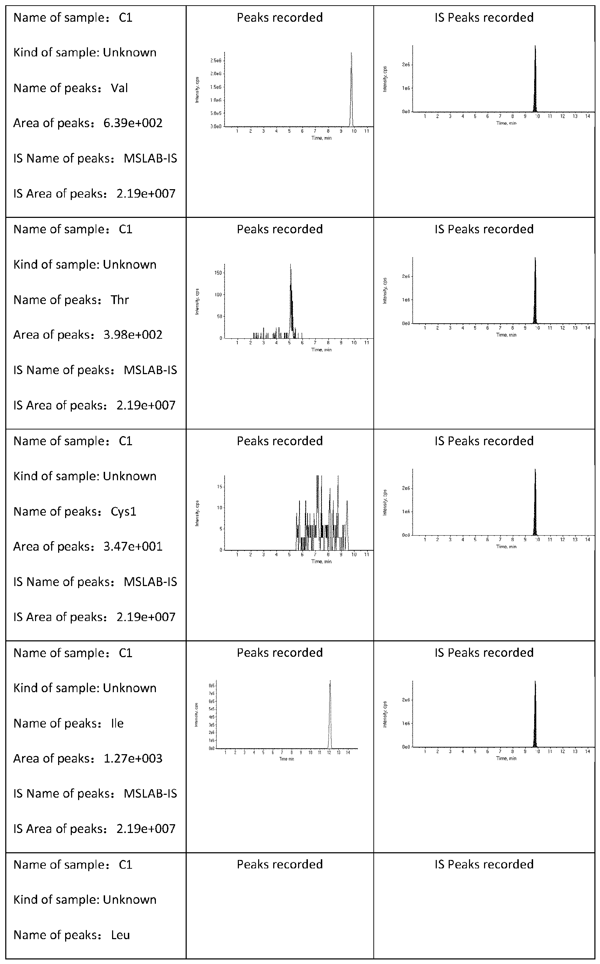

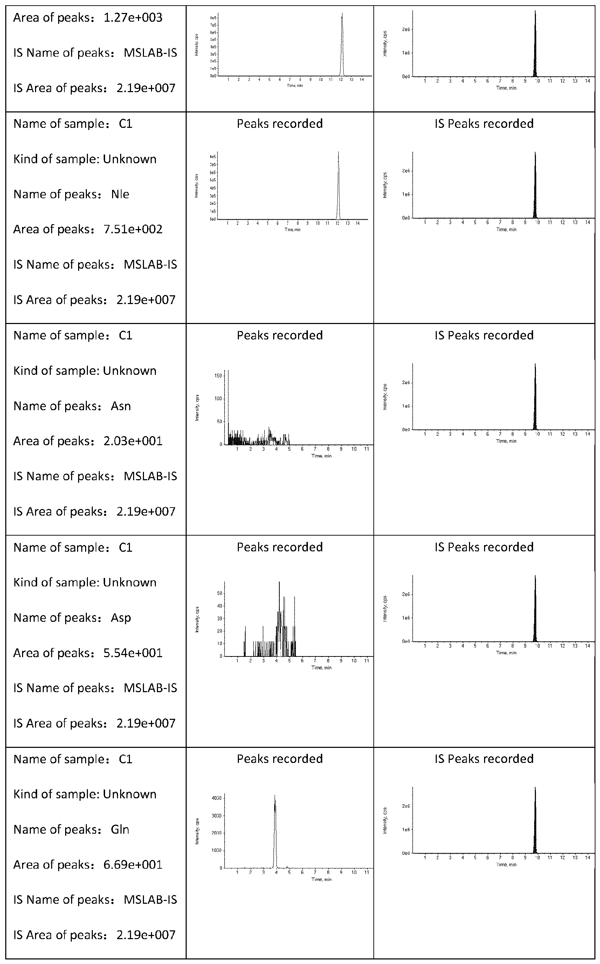

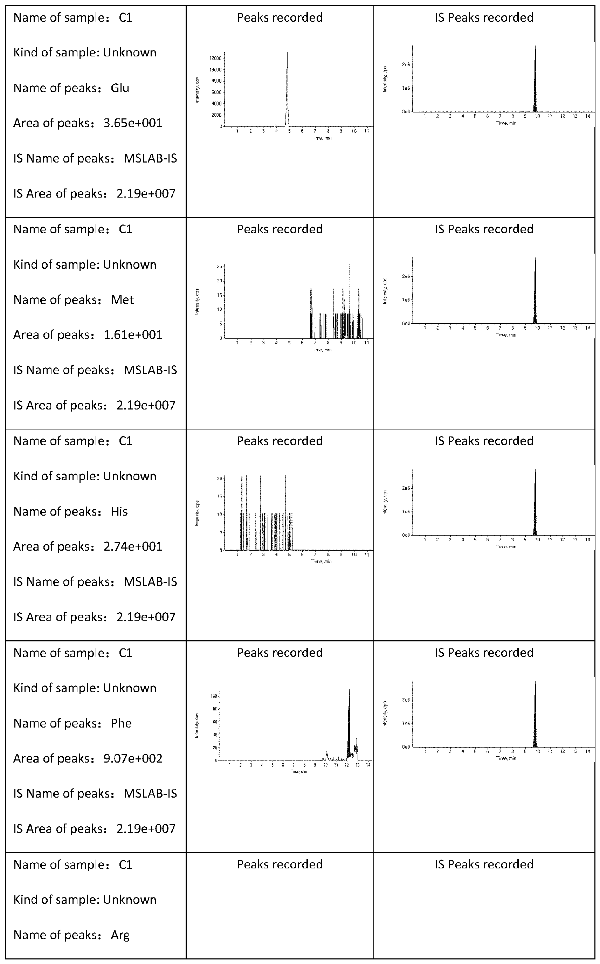

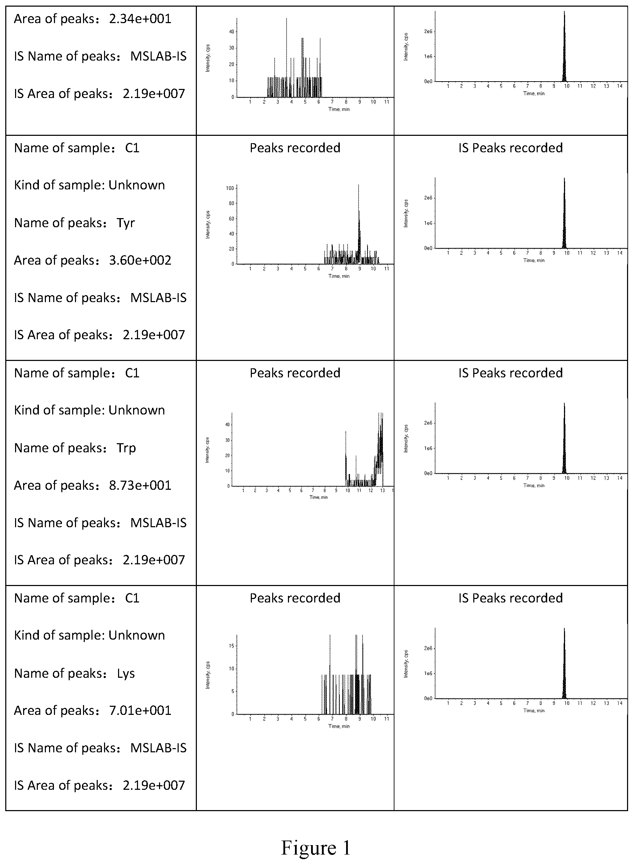

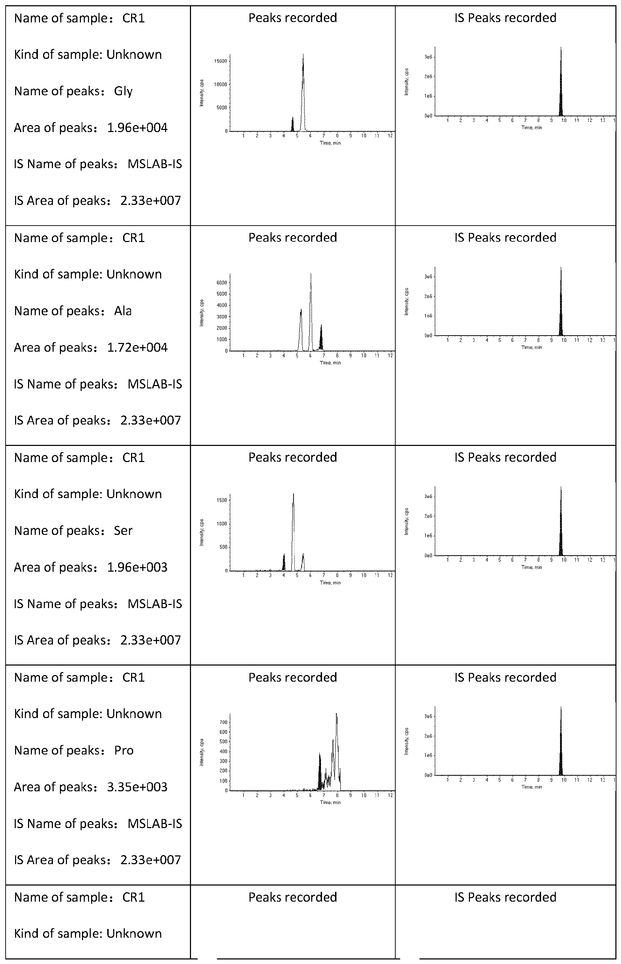

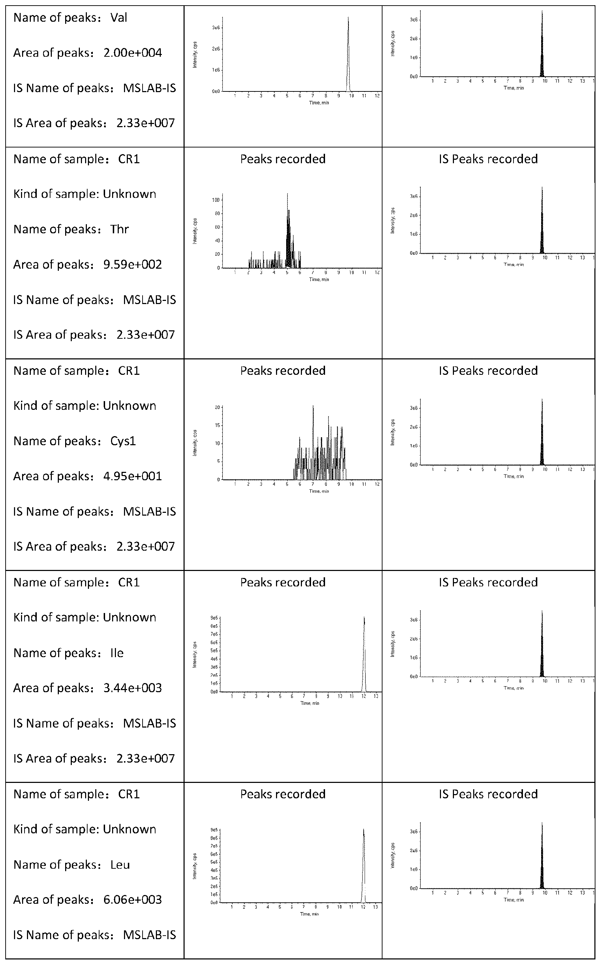

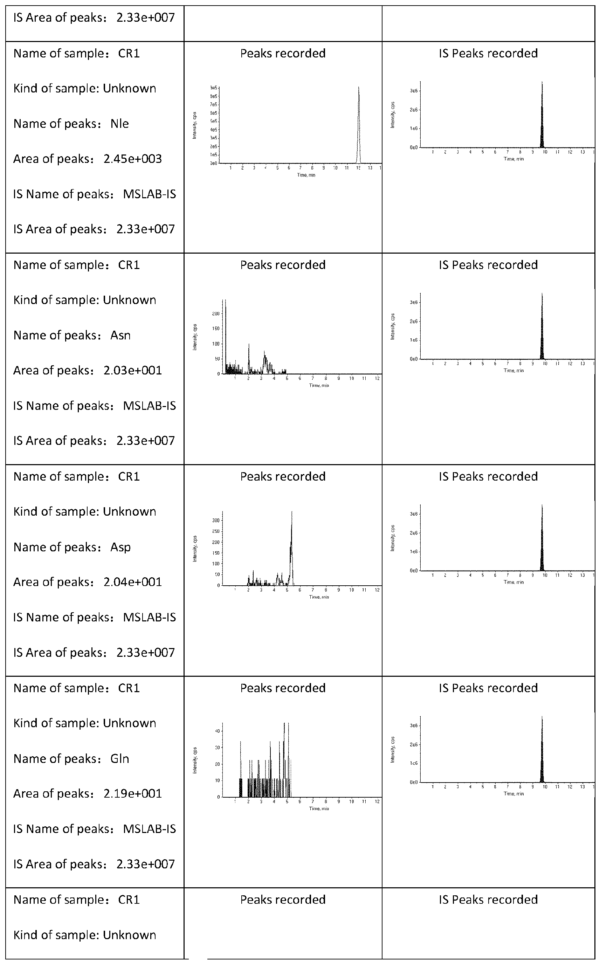

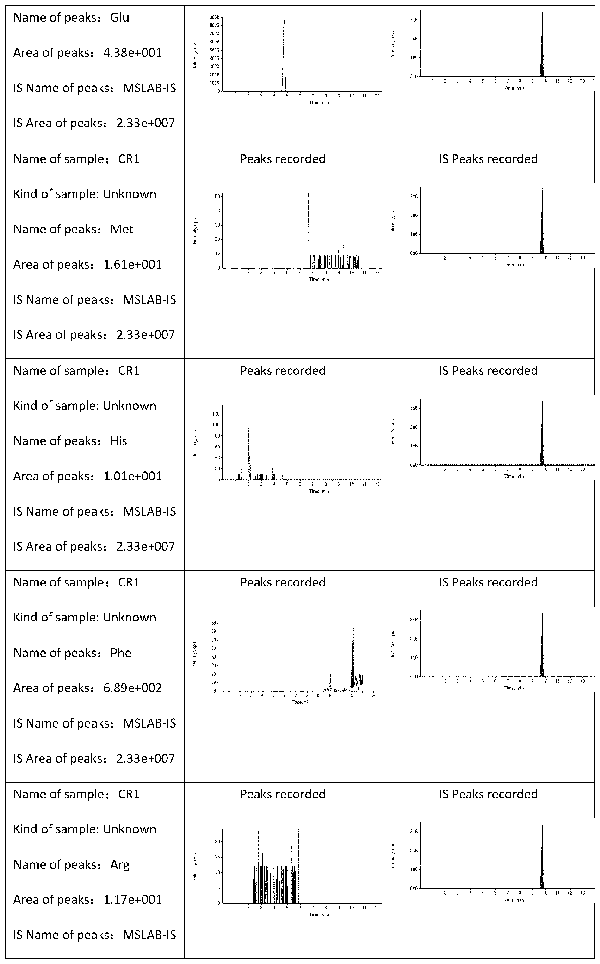

Fig. 1 illustrates an original spectrogram of chromatography-mass spectrometry of a product solution of Example 3 (reacted at 135°C±5°C by utilizing a Co catalyst and light irradiation).

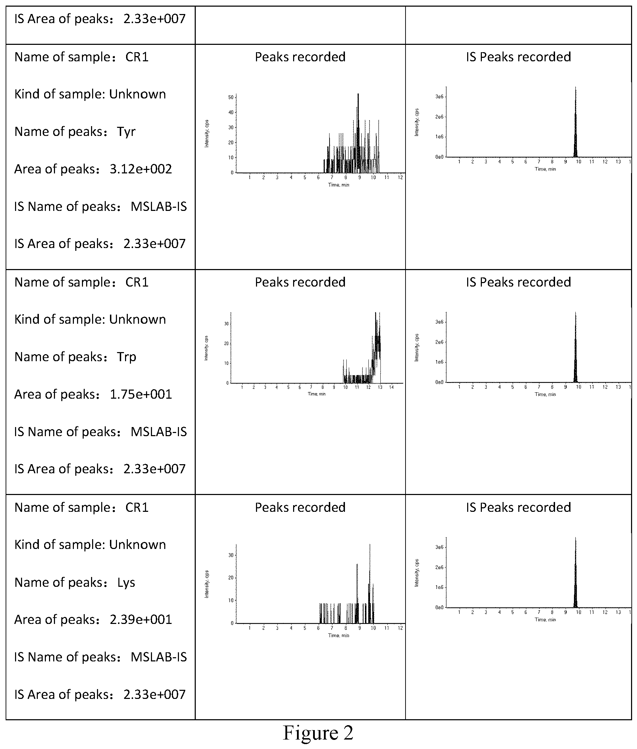

Fig. 2 illustrates an original spectrogram of chromatography-mass spectrometry of a product solution of Example 4 (reacted at 135°C±5°C by utilizing a Co-Ru catalyst and heat irradiation).

### EMBODIMENTS

The invention demonstrated, unexpected, that N₂, CO₂ and water could be converted to amino acids, using light irradiation and/or heat irradiation as energy input, in the presence of a nanostructure catalyst having plasmon effect.

Before further description of the present invention, certain terms employed in the specification, examples and appended claims are defined in the following section. The definitions listed herein should be read in light of the remainder of the disclosure and understood as by a person of skill in the art. Unless defined otherwise, all technical and scientific terms used herein have the same meaning as commonly understood by a person of ordinary skill in the art to which this invention belongs.

### DEFINITIONS

The term "catalyst" used herein refers to substances that exhibit effects to increase the rate of a chemical reaction by reducing the activation energy of the reaction. The rate increasing effect is referred as "catalysis". Catalysts are not consumed in a catalytic reaction, therefore they can continue to catalyze the reaction of further quantities of reactant with a small amount.

The term "plasmonic provider" used herein refers to conductor the real part of whose dielectric constant is negative. Plasmonic providers provide surface plasmons when excited by electromagnetic irradiation.

The term "chemical element" used herein refers to chemical substance consisting of atoms having the same number of protons in their atomic nuclei. Specifically, chemical elements are those recorded in the periodic table of chemical elements. Chemical elements include natural elements and synthetic elements. Chemical elements also include elements not discovered yet with more than 118 protons in the atomic nuclei.

The term "alloy" used herein refers to a mixture of metals or a mixture of metal and other elements. Alloys are defined by metallic bonding character. An alloy may be a solid solution of metal elements (a single phase) or a mixture of metallic phases (two or more phases).

The term "close contact" used herein refers to that there is basically no gap between the two components, e.g., a distance between the two components is equal to or less than one tenth, equal to or less than one twentieth of length, width or height of the nanostructure, or 1 nm or less, 0.1 nm or less, or basically 0.

The term "C₁₋₄ hydrocarbons" used herein includes C₁, C₂, C₃ and C₄ hydrocarbons, such as methane, ethane, n-propane, isopropane, n-butane, and isobutane.

The terms "substantially consisting of ..." used herein refers to that a ratio of the enumerated components in the composition is not less than 80%, preferably not less than 85%, not less than 90%, not less than 98%, not less than 99%, not less than 99.5%, the percentage could be mole percentage or weight percentage.

### NANOSTRUCTURE CATALYST

One aspect of the present invention is a nanostructure catalyst used in a reaction for producing amino acids by means of energy irradiation.

Without wishing to be bound by theory, the nanostructure catalyst of the present invention interacts with the raw materials of the reaction to reduce the activation energy of the reaction so as to initiate the reaction by means of energy irradiation, and to increase the reaction rate.

The nanostructure catalyst of the present invention comprises two components: a first component and a second component. One component is plasmonic provider, and the other component is catalytic property provider. The plasmonic provider provides surface plasmon resonance enhancement to the localized field on catalysts, and the catalytic property provider provides catalytic property to the reaction that produces amino acid. Different plasmonic providers have different plasmon enhancement strength and active life time. For example, noble metal elements such as Ru and Pd have high plasmon enhancement strength and long active life time. Different catalytic property providers also have different catalytic strength and active life time. For example, Cu and Ni have middle catalytic strength but longer active life time. Elements such as Co, Fe and their oxides have high catalytic strength but short active life time. Metal carbides have relatively good catalytic strength, but short active life time.

In the nanostructure catalyst of the present invention, a distance between the first component and the second component is 200 nm or less, preferably 100 nm or less, and most preferably the first component and the second component are in close contact with each other. If the distance between the first component and the second component is out of the above range, both components could not interact with each other, and thus the energy irradiation reaction of the present invention could not be catalyzed.

The first component is a conductor the real part of whose dielectric constant is negative. It could be a pure substance (chemical element) or an alloy, and it may be selected from a group consisting of Co, Fe, Al, Ag, Au, Pt, Cu, Ni, Zn, Ti, Mn, C, Pd, Ru and alloys of two or more chemical elements thereof. From the viewpoint of efficiency, Ru, Co or Fe is most preferred in the present invention.

The second component may be a pure substance (chemical element) or a compound, and it may be selected from a group consisting of Co, Fe, Ru, Rh, Os, Ir, La, Ce, Cu, Ni, Ti, Mo, Bi, V, C and oxides, sulfites, carbides, hydroxides, chlorides, carbonates and bicarbonates thereof. Co, C or MnO₂ is most preferred in the present invention.

The first component and the second component can be randomly mixed, or regularly mixed. In some embodiments, the nanostructure catalyst comprises one chemical element as both the first component and the second component, or comprises two or more chemical elements, alloys, or compounds each as the first component or the second component. In some embodiments, the nanostructure catalyst is produced by physically mixing the at least one first component and the at least one second component. In preferred embodiments, the at least one first component and the at least one second component of the nanostructure catalyst are provided in one nanostructure, e.g., the first component and the second component form an alloy. Specifically, the nanostructure catalyst could be nanoparticles of the aforementioned elements, or nanoparticles of the alloys of the aforementioned elements, as long as the nanoparticles could provide both the plasmonic property and the catalytic property. In preferred embodiments, the first component plays a role as the plasmonic provider, and the second component plays a role as the catalytic property provider.

As can be seen from the above description, certain elements are capable of exhibiting both the plasmonic property and the catalytic property. Accordingly, the plasmonic provider and the catalytic property provider of the nanostructure catalyst could be a same element, such as Co, Fe, Cu, Ni, C, Ru and Ti, etc, or could be the element and oxide, chloride, carbonate and bicarbonate thereof, such as Co and CoO, Fe and FeO, etc.

Mixture of different elements will modify the property of these plasmonic nanoparticle catalysts. For example, Co/Ru alloy would increase the active life time and catalytic effects of the catalysts; Co/C would solely increase the effect of the catalyst, but reduce the active life time. In the present invention, the nanostructure catalyst is preferably Co/Ru, Co, Fe/C or Fe/C/MnO₂, which exhibit good active life time and catalytic effects for catalytically producing amino acid, such as yield of amino acids. When the nanostructure catalyst is a Co-Cu catalyst, a mole ratio of Co to Ru in the Co-Ru catalyst is 10:1 to 2000:1, preferably 100:1 to 1200:1, more preferably 120:1 to 600:1, and most preferably 150:1 to 300:1.

### NANOSTRUCTURE

The term "nanostructure" used herein refers to a structure having at least one dimension within nanometer range, i.e. about 1 nm to about 1000 nm, preferably about 70 nm to about 1000 nm, about 100 nm to about 800 nm, about 200 nm to about 500 nm in at least one of its length, width, and height. Nanostructure can have one dimension which exceeds 1000 nm, for example, having a length in micrometer range such as 1 µm to 5 µm. In certain cases, tubes and fibers with only two dimensions within nanometer range are also considered as nanostructures. Material of nanostructure may exhibit size-related properties that differ significantly from those observed in bulk materials.

The nanostructure of the present invention each independently is about 1 nm to about 3000 nm in length, width or height. The length thereof is preferably about 100 nm to about 3000 nm, more preferably about 500 nm to about 2500 nm, and yet more preferably about 1000 nm to about 2000 nm. The width or height thereof is preferably about 1 nm to about 1000 nm, preferably about 70 nm to about 1000 nm, more preferably about 100 nm to about 800 nm, and yet more preferably about 200 nm to about 500 nm.

The nanostructure of the present invention each independently has an aspect ratio of about 1 to about 20 (i.e., a ratio of length to width/height), preferably an aspect ratio of about 1 to about 10, or about 2 to about 8. The nanostructure of the present invention can also have a relatively low aspect ratio such as about 1 to about 2.

The nanostructure of the present invention each independently has a shape of spherical, spike, flake, needle, grass, cylindrical, polyhedral, 3D cone, cuboidal, sheet, hemispherical, irregular 3D shape, porous structure or any combinations thereof.

A plurality of the nanostructures of the present invention can be arranged in a patterned configuration, preferably in a plurality of layers, on a substrate, or randomly dispersed in a medium. For example, nanostructures may be bound to a substrate. In such case, the nanostructures are generally not aggregated together, but rather, pack in an orderly fashion. Alternatively, a plurality of nanostructures can be dispersed in a liquid medium, in which each nanostructure is free to move with respect to any other nanostructures.

For example, the nanostructure could have a spike or grass-like geometric configuration. Optionally, the nanostructure has a geometric configuration with a relatively thin thickness. Preferably, the nanostructure has a configuration of nano-jungle, nano-grass, and/or nano-snowflake. The nanostructure could have a relatively large aspect ratio, such nanostructure could have a construction of nano-spike, nano-snowflake or nano-needle. The aspect ratio could be about 1 to about 20, about 1 to about 10, or about 2 to about 8. Preferably, the length of the nanostructure could be about 100 nm to about 3000 nm, about 500 nm to about 2500 nm, or about 1000 nm to about 2000 nm; the width or the height could be about 1 nm to about 1000 nm, about 70 nm to about 1000 nm, about 100 nm to about 800 nm, or about 200 nm to about 500 nm.

The nanostructures may be bound to a substrate. Accordingly, the nanostructures are generally not aggregated together, but rather, pack in an orderly fashion. The substrate could be formed of metal or polymer material (e.g., polyimide, PTFE, polyester, polyethylene, polypropylene, polystyrene, polyacrylonitrile, etc.)

In other embodiments, the nanostructure has a shape of spherical, spike, cylindrical, polyhedral, 3D cone, cuboidal, sheet, hemispherical, irregular 3D shape, porous structure or any combinations thereof. Such nanostructures each independently is about 1 nm to about 1000 nm, preferably about 70 nm to about 1000 nm, about 100 nm to about 800 nm, or about 200 nm to about 500 nm in length, width or height. The plasmonic provider and the catalytic property provider can be randomly mixed or regularly mixed. A distance between the plasmonic provider and the catalytic property provider is less than 200 nm, preferably less than 100 nm, and more preferably that the plasmonic provider and the catalytic property provider are in close contact with each other. In preferred embodiments, the two components are provided in one nanostructure, e.g., the nanostructure is alloy of two or more chemical elements.

Furthermore, the nanostructure catalyst of the present invention could function in various states, such as dispersed, congregated, or attached/grown on surface of other materials. In preferred embodiments, the nanostructures are dispersed in a medium, and the medium is preferably the reactants of the reaction, such as water.

### METHOD FOR PRODUCING AMINO ACIDS

Another aspect of the present invention is a method for producing amino acids by energy irradiation, comprising:
contacting the aforementioned nanostructure catalyst with at least one nitrogen-containing source, at least one hydrogen-containing source and at least one carbon-containing source, and
irradiating the nanostructure catalyst, the nitrogen-containing source, the hydrogen-containing source and the carbon-containing source with energy, to produce amino acids.

Under catalytic effects of the nanostructure catalyst, the energy irradiation, i.e., light irradiation and/or heat irradiation, initiates the reaction of the nitrogen-containing source, the hydrogen-containing source and the carbon-containing source. Without wishing to be bound by theory, the nanostructure catalyst of the present invention could convert and transmit energy of the light irradiation and the heat irradiation, so as to keep the reaction of the present invention progressing.

The energy irradiation allows the reaction progresses at a temperature between about 20°C to about 300°C, preferably about 30°C to about 300°C, about 40 °C to about 300 °C, about 80 °C to about 300°C, about 100°C to about 280°C, about 110°C to about 270°C.
at a temperature between about 20°C to about 300°C, the reaction could produce amino acids comprising isoleucine, leucine, phenylalanine, lysine, threonine and valine, and preferably optionally comprising methionine and tryptophan.

The term "heat" used herein refers to thermal energy transferred from one system to another as a result of thermal exchanges. Heat may be transferred into the reaction system with an external heat source, alternatively, heat may be inherently carried by one component of the reaction so as to be transferred to other components involved in the reaction. In other words, the one component that inherently carries heat before reaction is an internal heat source. In certain embodiments, the temperature of the nanostructure catalyst, the nitrogen-containing source, the hydrogen-containing source and the carbon-containing source is raised by heat irradiation.

In the reaction of the present invention, the light irradiation mimics the wavelength composition and the strength of solar light, thus the temperature of the irradiated catalyst and reaction mixture could be raised. When the irradiation intensity reaches a certain level, the temperature of the nanostructure catalyst, the nitrogen-containing source, the hydrogen-containing source and the carbon-containing source is raised by the light irradiation.

In the reaction of the present invention, the temperature of nanostructure catalyst, the nitrogen-containing source, the hydrogen-containing source and the carbon-containing source is raised by the light irradiation, and preferably the light irradiation is the sole source for rising the temperatures.

In the reaction of the present invention, after the reaction is initiated, the reaction is continued to progress under light irradiation. The term "light" used herein refers to electromagnetic wave having a wavelength from about 250 nm to about 2000 nm. In other words, light refers to the irradiance of visible light. Preferably, in the reaction of the present invention, a power of light irradiation is less than the power of solar irradiation (i.e., the solar constant). For example, the power of light irradiation is 200-1500 W/m², preferably 200-1000 W/m², and most preferably 500-1000 W/m². The light irradiation could be solar light or light emitted from artificial light sources, wavelength of the light irradiation is between about 250 nm to about 2000 nm.

The reaction period varies depending on reaction scale, irradiation intensity, temperature and other factors, the reaction is continuously performed with a continuous feed of the nitrogen-containing source, the hydrogen-containing source and the carbon-containing source with a well-established apparatus. The reaction period could be 0.1 hour or more, preferably 0.1 hour to 1000 hours, preferably 0.1 hour to 500 hours, preferably 0.5 hour to 100 hours, preferably 1 hour to 50 hours, preferably 2 hours to 30 hours, and most preferably 4 hours to 20 hours.

The reaction could be carried out at low pressure, normal pressure or high pressure, the reaction pressure could be properly selected based on reaction scale, irradiation intensity, temperature and other factors, for example, the reaction pressure could be at least 1 bar, e.g. 1 bar to 30 bar, preferably 1 bar to 20 bar, and more preferably 1.5 bar to 5 bar.

### REACTION MATERIALS

In the reaction of the present invention, reaction materials include the nitrogen-containing source, the hydrogen-containing source and the carbon-containing source.

The nitrogen-containing source is selected from a group consisting of N₂, air, ammonia, nitrogen oxides, nitro compounds and any combination thereof, or air, industrial flue gas, exhausts or emissions comprising one or more of these nitrogen-containing sources. In the reaction of the present invention, the nitrogen-containing source is preferably N₂.

The hydrogen-containing source is selected from a group consisting of water, H₂, C₁₋₄ hydrocarbons and any combination thereof, or air, industrial flue gas, exhausts or emissions comprising one or more of these hydrogen-containing sources. In the reaction of the present invention, the hydrogen-containing source is preferably water, and water could be in gaseous state or liquid state.

The carbon-containing source is selected from a group consisting of CO₂, CO, C₁₋₄ hydrocarbons, synthesis gas, bicarbonate salts and any combination thereof, or air, industrial flue gas, exhausts or emissions comprising one or more of these carbon-containing sources. In the reaction of the present invention, the carbon-containing source is preferably CO₂ and CO, and more preferably CO₂.

As can be seem from the above reaction materials, the nitrogen-containing source, the hydrogen-containing source and the carbon-containing source that could be utilized in the present invention are contained widely in industrial exhaust gas, waste water, flue gas, combustion emission and automobile exhaust, which could be used as the reaction materials of the present invention, so as to promote recycling and treatment of industrial wastes.

### REACTION PRODUCTS

Amino acids could be produced by the reaction of the present invention. Without wishing to be bound by theory, decomposition and recombination of various reaction materials molecules on nanostructures of the plasmonic metal catalysts could be included in the reaction mechanism of the present invention.

### EXAMPLES

### Example 1 Preparation of the nanostructure catalyst

The Co catalyst was prepared by following method:
8.1 g of cobalt chloride hexahydrate (CoCl₂·6H₂O) was added into a 250 mL three-necked flask, dissolved by adding 100 mL water under stirring, heated to 80°C and then refluxed;
10.88 g of sodium hydroxide (NaOH) was dissolved in 30 mL water, 30 mL of hydrazine hydrate (N₂H₄·H₂O) was added, and then the liquid mixture was slowly added, dropwise, into the three-necked flask with a speed of 1.8 mL min⁻¹ under control of a titration pump. After the reaction was conducted for 60 min, the solution was cooled down to room temperature, centrifugal separated at 5000 rpm for 5 min and ultrasonic washed with deionized water for 3 times, and dried at 70°C in a vacuum oven, so as to obtain the Co catalyst to be used in experiments. After characterized by SEM, the Co catalyst showed morphology of nanoparticles having a particle diameter of about 100 nm.

The Co-Ru catalyst was prepared by following method:
8.1 g of cobalt chloride hexahydrate (CoCl₂·6H₂O) and 0.042 g of ruthenium trichloride (RuCl₃·xH₂O) were added into a 250 mL three-necked flask, dissolved by adding 100 mL water under stirring, heated to 80°C and then refluxed;10.88 g of sodium hydroxide (NaOH) was dissolved in 30 mL water, 30 mL of hydrazine hydrate (N₂H₄·H₂O) was added, and then the liquid mixture was slowly added, dropwise, into the three-necked flask with a speed of 1.8 mL min⁻¹ under control of a titration pump. After the reaction was conducted for 60 min, the solution was cooled down to room temperature, centrifugal separated at 5000 rpm for 5 min and ultrasonic washed with deionized water for 3 times, and dried at 70°C in a vacuum oven,
so as to obtain the Co-Ru catalyst to be used in experiments. After characterized by SEM, the Co-Ru catalyst showed morphology of nanoparticles having a particle diameter of about 200 nm, including Co nanoparticles, Ru nanoparticles and Co-Ru alloy nanoparticles.

The Fe/C catalyst and the Fe/C/MnO₂ catalyst were prepared by following method:
the Fe catalysts were prepared via similar method as that for producing the Co catalyst. After characterized by SEM, the Fe catalyst showed morphology of Fe nanoparticles having a particle diameter of about 100 nm.

The prepared Fe catalyst was mixed with C powder and MnO₂ powder in specific mass ratios, to obtain the Fe/C (9:1) catalyst and the Fe/C/MnO₂ (8:1:1) catalyst. Both the carbon (graphite powder, 99.95%, 100 meshes, CAS: 7782-42-5) and the MnO₂ (99%, CAS: 1313-13-9) powder were purchased from Aladdin.

Example 2 Photocatalytic reaction for producing amino acids using the Co catalyst 2 g of Co catalyst and 6 mL ultrapure water were added into a sealable pressure-bearing glass tube having a volume of 35 mL, 2 bar N₂ and 2 bar CO₂ were filled into the glass tube after air in the glass tube was replaced with N₂. The glass tube was laid flat on glass wool, the catalyst and water were tiled, and a halogen lamp with controlled voltage was employed to irradiate vertically from the above. The intensity of the incident light was about 1000 W/m². Thermocouples were connected to the lower part of the glass tube to monitor the temperature. The temperature of the glass tube was controlled to 100°C±5°C, the irradiation was continued for 18 h to conduct the photocatalytic reaction.

After the photocatalytic reaction, the solution of the reaction tube was cooled down to room temperature by standing, centrifugal separated to obtain a supernatant. Quantitative analysis of amino acids in the sample was conducted using high performance liquid chromatography-mass spectrometry (LC liquid chromatograph: Dionex Ultimate 3000; MS mass spectrometer: Applied Biosystems API 3200 Q TRAP), the amino acid kit MSLAB50AA with a batch number of MSLAB50AA170601# was purchased from Beijing Mass Spectrum Medical Research CO., LTD.

A decline in catalytic activity of the Co catalyst for producing amino acids was not found after continuously reacted for 80 h.

Example 3 Photocatalytic reaction for producing amino acids using the Co catalyst The photocatalytic reaction and the quantitative analysis were performed in the same manner as Example 2, the reaction temperature was controlled to 135°C±5°C, and the light irradiation was continuously conducted for 18 h.

A decline in catalytic activity of the Co catalyst for producing amino acids was not found after continuously reacted for 80 h.

### Example 4 Thermal catalytic reaction for producing amino acids using the Co-Ru catalyst

2 g of Co-Ru catalyst and 6 mL ultrapure water were added into a sealable pressure-bearing glass tube having a volume of 35 mL, 2 bar N₂ and 2 bar CO₂ were filled into the glass tube after air in the glass tube was replaced with N₂. The glass tube was wrapped with aluminum foil to be isolated from light irradiation. The glass tube was placed in a temperature-controlled oven, and heated to initiate the reaction. The temperature in the oven was controlled to 135°C ±5°C, and the reaction was continued for 18 h.

The solution obtained from the reaction was quantitatively analyzed in the same manner as Example 2.

A decline in catalytic activity of the Co-Ru catalyst for producing amino acids was not found after continuously reacted for 80 h.

Example 5 Thermal catalytic reaction for producing amino acids using the Fe/C (9:1) catalyst The thermal catalytic reaction and the quantitative analysis were performed in the same manner as Example 4 utilizing the Fe/C (9:1) catalyst, the temperature in the oven was controlled to 100°C±5°C, and the reaction was continuously conducted for 18 h.

A decline in catalytic activity of the Fe/C (9:1) catalyst for producing amino acids was not found after continuously reacted for 80 h.

### Example 6 Thermal catalytic reaction for producing amino acids by utilizing the Fe/C/MnO₂ (8:1:1) catalyst

The thermal catalytic reaction and the quantitative analysis were performed in the same manner as Example 4 utilizing the Fe/C/MnO₂ (8:1:1) catalyst, the temperature in the oven was controlled to 135°C±5°C, and the reaction was continuously conducted for 18 h.

A decline in catalytic activity of the Fe/C/MnO₂ (8:1:1) catalyst for producing amino acids was not found after continuously reacted for 80 h.

Kinds and contents (µmol/L) of the amino acids produced in Example 2 to 6 were shown in table 1.

**Table 1: Kinds and contents (µmol/L) of the amino acids produced in Example 2 to 6**

| Kinds of amino acids | Example 2 Co about 100°C photocatalytically | Example 3 Co about 135°C photocatalytically | Example 4 Co-Ru about 135°C thermal catalytically | Example 5 Fe/C (9:1) about 100°C thermal catalytically | Example 6 Fe/C/MnO₂ (8:1:1) about 135°C thermal catalytically |
|---|---|---|---|---|---|
| glycine (Gly) | 0.130 | 0.178 | 0.402 | 0.692 | 0.333 |
| alanine (Ala) | 0.375 | 0.478 | 0.613 | 0.322 | 0.331 |
| serine (Ser) | 0.378 | 0.291 | 0.107 | 0.001 | 0.002 |
| proline (Pro) | 0.159 | 0.198 | 0.078 | 0.079 | 0.079 |
| valine (Val) | 0.091 | 0.103 | 0.327 | 0.028 | 0.036 |
| threonine (Thr) | 0.108 | 0.117 | 0.028 | 0.086 | 0.125 |
| cysteine (Cys) | 0.158 | 0.222 | 0.183 | 0.151 | 0.168 |
| isoleucine (Ile) | 0.410 | 0.400 | 0.409 | 0.388 | 0.395 |
| leucine (Leu) | 0.254 | 0.242 | 0.263 | 0.216 | 0.228 |
| asparagine (Asn) | 0.014 | 0.017 | 0.005 | 0.034 | 0.006 |
| aspartic acid (Asp) | 0.022 | 0.032 | 0.001 | 0.107 | 0.119 |
| glutamine (Gln) | 0.147 | 0.360 | 0.002 | 0.024 | 0.013 |
| glutamate (Glu) | 0.024 | 0.065 | 0.015 | 0.015 | 0.018 |
| methionine (Met) | 0.018 | 0.023 | 0.000 | 0.005 | 0.000 |
| phenylalanine (Phe) | 0.255 | 0.250 | 0.222 | 0.243 | 0.248 |
| tyrosine (Tyr) | 0.051 | 0.043 | 0.010 | 0.030 | 0.046 |
| tryptophan (Trp) | 0.032 | 0.020 | 0.000 | 0.008 | 0.011 |
| lysine (Lys) | 0.307 | 0.109 | 0.008 | 0.020 | 0.061 |

As can be seen from the results of Examples 2 to 6, in a case of the overall reaction conditions were mild, under the heat irradiation and/or the light irradiation with a relative low intensity, and in a case of the reaction temperature was lower than 300°C, the method of the present application could highly effectively convert readily available raw materials into amino acids products with high value, the amino acids products comprise amino acids essential to human body.

In this specification and the appended claims, the singular forms "a", "an", and "the" include plural reference, unless the context clearly indicates otherwise. Unless defined otherwise, all technical and scientific terms used herein have the same meaning as commonly understood to one of ordinary skill in the art. Methods recited herein may be carried out in any order that is logically possible, in addition to a particular order disclosed.

The representative examples are intended to help illustrate the invention, and are not intended to, nor should they be construed to, limit the scope of the invention. Indeed, various modifications of the invention and many further embodiments thereof, in addition to those shown and described herein, will become apparent to those skilled in the art from the full contents of this document, including the examples and the references to the scientific and patent literature included herein. The examples contain important additional information, exemplification and guidance that can be adapted to the practice of this invention in its various embodiments and equivalents thereof.

## Claims

1. An amino acid composition comprising amino acids essential to human body, comprising isoleucine, leucine, phenylalanine, lysine, threonine and valine, and preferably optionally comprising methionine and tryptophan.

2. The amino acid composition according to claim 1, wherein the amino acid composition comprises 0.36-0.44 part by mole of isoleucine, 0.20-0.28 part by mole of leucine, 0.20-0.27 part by mole of phenylalanine, 0.008-0.32 part by mole of lysine, 0.02-0.14 part by mole of threonine, 0.02-0.35 part by mole of valine, 0-0.04 part by mole of methionine and 0-0.04 part by mole of tryptophan.

3. The amino acid composition according to claim 1 or 2, wherein the amino acid composition further comprises glycine, alanine and cysteine.

4. The amino acid composition according to claim 3, wherein the amino acid composition comprises 0.12-0.71 part by mole of glycine, 0.30-0.62 part by mole of alanine and 0.14-0.23 part by mole of cysteine.

5. The amino acid composition according to any one of claims 1 to 4, wherein the amino acid composition further comprises serine, glutamine, proline, asparagine, aspartic acid, glutamate and tyrosine, the composition preferably comprises water as solvent.

6. The amino acid composition according to claim 1 or 2, wherein the amino acid composition is substantially consisting of isoleucine, leucine, phenylalanine, lysine, threonine, valine, methionine and tryptophan.

7. A method for producing amino acids by energy irradiation, comprising:
contacting a nanostructure catalyst with at least one nitrogen-containing source, at least one hydrogen-containing source and at least one carbon-containing source, and
irradiating the nanostructure catalyst, the nitrogen-containing source, the hydrogen-containing source and the carbon-containing source with energy, to produce amino acids, wherein
the nanostructure catalyst comprises at least one first component and at least one second component.

8. The method according to claim 7, wherein
the energy irradiation is at least one selected from light irradiation and heat irradiation.

9. The method according to claim 7 or 8, wherein
a distance between the first component and the second component is 200 nm or less, preferably 100 nm or less, and most preferably the first component and the second component are in close contact with each other.

10. The method according to any one of claims 7 to 9, wherein
the nanostructure catalyst comprises one chemical element as both the first component and the second component, or comprises two or more chemical elements, alloys, or compounds each as the first component or the second component.

11. The method according to any one of claims 7 to 10, wherein
the nanostructure catalyst is produced by physically mixing the at least one first component and the at least one second component.

12. The method according to any one of claims 7 to 10, wherein
the nanostructure catalyst provides the at least one first component and the at least one second component in one nanostructure.

13. The method according to any one of claims 7 to 12, wherein
the first component is selected from a group consisting of Co, Fe, Al, Ag, Au, Pt, Cu, Ni, Zn, Ti, Mn, C, Pd, Ru and alloys of two or more chemical elements thereof, and preferably Ru, Co, Fe or C.

14. The method according to any one of claims 7 to 13, wherein
the second component is selected from a group consisting of Co, Fe, Ru, Rh, Os, Ir, La, Ce, Cu, Ni, Ti, Mo, Bi, V, C and oxides, sulfites, carbides, hydroxides, chlorides, carbonates and bicarbonates thereof, and preferably Co, C or MnO₂.

15. The method according to any one of claims 7 to 14, wherein
the nanostructure catalyst is a Co catalyst, a Co-Ru catalyst, a Fe/C catalyst or a Fe/C/MnO₂ catalyst.

16. The method according to any one of claims 7 to 15, wherein
the amino acid compositions according to claims 1-6 are produced by the method.

17. The method according to any one of claims 7 to 16, wherein
the nanostructure is about 1 nm to about 1000 nm, preferably about 70 nm to about 1000 nm, about 100 nm to about 800 nm, about 200 nm to 500 nm in at least one dimension of length, width and height.

18. The method according to any one of claims 7 to 17, wherein
the nanostructure each independently is about 1 nm to about 3000 nm in length, width or height, preferably is about 100 nm to about 3000 nm, about 500 nm to about 2500 nm, or about 1000 nm to about 2000 nm in length, and/or about 1 nm to about 1000 nm, about 70 nm to about 1000 nm, about 100 nm to about 800 nm, or about 200 nm to about 500 nm in width or height, or
the nanostructure each independently has an aspect ratio of about 1 to about 20, and preferably an aspect ratio of about 1 to about 10, or about 2 to about 8.

19. The method according to any one of claims 7 to 18, wherein
the nanostructure catalyst each independently has a shape of spherical, spike, flake, needle, grass, cylindrical, polyhedral, 3D cone, cuboidal, sheet, hemispherical, irregular 3D shape, porous structure or any combinations thereof.

20. The method according to any one of claims 7 to 19, wherein
a plurality of the nanostructures are arranged in a patterned configuration, and preferably in a plurality of layers, on a substrate, or
a plurality of the nanostructure are randomly dispersed in a medium.

21. The method according to any one of claims 7 to 20, wherein
the energy irradiation allows the reaction progresses at a temperature between about 20°C to about 300°C, preferably about 30°C to about 300°C, about 40 °C to about 300 °C, about 80 °C to about 300°C, about 100°C to about 280°C, about 110°C to about 270°C.

22. The method according to any one of claims 8 to 21, wherein
the reaction was initiated by means of light irradiation or heat irradiation, and the reaction is continued to progress by means of light irradiation or heat irradiation, wherein
a power of the light irradiation is 200-1500 W/m², preferably 200-1000 W/m², and most preferably 500-1000 W/m².

23. The method according to any one of claims 8 to 22, wherein
the temperature of the nanostructure catalyst, the nitrogen-containing source, the hydrogen-containing source and the carbon-containing source is raised by the light irradiation, and preferably the light irradiation is the sole source for raising the temperature.

24. The method according to any one of claims 7 to 23, wherein
the nitrogen-containing source is selected from a group consisting of N₂, air, ammonia, nitrogen oxides, nitro compounds and any combination thereof, or air, industrial flue gas, exhausts or emissions comprising one or more of these nitrogen-containing sources, and preferably N₂.

25. The method according to any one of claims 7 to 24, wherein
the hydrogen-containing source is selected from a group consisting of water, H₂, C₁₋₄ hydrocarbons and any combination thereof, or air, industrial flue gas, exhausts or emissions comprising one or more of these hydrogen-containing sources, and preferably water.

26. The method according to any one of claims 7 to 25, wherein
the carbon-containing source is selected from a group consisting of CO₂, CO, C₁₋₄ hydrocarbons, synthesis gas, bicarbonate salts and any combination thereof, or air, industrial flue gas, exhausts or emissions comprising one or more of these carbon-containing sources, and preferably CO₂ or CO.
